# EUROPEAN PATENT APPLICATION

(11) **EP 4 613 221 A1**
(43) Date of publication of application: **10.09.2025**
(21) Application number: 22964333.3
(22) Date of filing: 31.10.2022
(51) Int. Cl.: A61B 17/56

(54) **NAVIGATION SYSTEM, JIG, AND PROGRAM**

(71) Applicant: Tohoku University, Sendai-shi, Miyagi 980-8577 (JP)
(72) Inventor: YAMAUCHI Kensuke, Sendai-shi, Miyagi 980-8577 (JP); HARIYAMA Masanori, Sendai-shi, Miyagi 980-8577 (JP); YODA Nobuhiro, Sendai-shi, Miyagi 980-8577 (JP)
(74) Representative: Plasseraud IP
(86) International application number: PCT/JP2022/040624
(87) International publication number: WO 2024/095310

(57) **Abstract**

A navigation system includes: an acquisition unit configured to acquire information on a position and a posture of a first sensor detected based on a signal of the first sensor, and information on a target attachment position and a target attachment posture of the first sensor with respect to a position and a posture of a second sensor detected based on a signal of the second sensor, the first sensor being inserted into an attachment portion of a first jig, which has a surface having a shape corresponding to a surface of a first bone, in a first orientation predetermined with respect to the first jig, and the second sensor being inserted into an attachment portion of a second jig, which has a surface having a shape corresponding to a surface of a second bone, in a second orientation predetermined with respect to the second jig; a derivation unit configured to derive a first relative position and a first relative posture of the first sensor based on the target attachment position, the target attachment posture, and the position and the posture of the first sensor; an information generation unit configured to generate a three-dimensional coordinate axis image representing the target attachment position and the target attachment posture and a three-dimensional coordinate axis image representing the first relative position and the first relative posture; and a display unit configured to display the three-dimensional coordinate axis image.

## Description

### Technical Field

The present invention relates to a navigation system, a jig, and a program.

### Background Art

As image processing technology advances, it has become common to perform preoperative planning (three-dimensional simulation) prior to surgery. For example, in orthognathic surgery, a surgeon may separate a maxilla (Le Fort I bone fragment) from a skull. The surgeon accurately moves the maxilla to a target relative position with respect to a position of the skull, and fixes the maxilla to the skull again at the target relative position.

In preoperative planning of such orthognathic surgery, a skull model and a maxilla model of a patient are moved and rotated on a screen of a display unit of a planning device (information processing device), and the target relative position of the maxilla with respect to an actual position of the skull is examined by a planner (doctor) for a purpose of achieving good occlusion and aesthetic facial appearance of the patient.

Navigation surgery is one of methods for reflecting a result of a target relative position of preoperative planning in actual surgery. In optical navigation surgery, a position of a surgical instrument is detected in real time by distance measurement using infrared rays reflected by a reflector attached to the surgical instrument.

Further, it has been studied to apply magnetic (electromagnetic (EM)) navigation surgery to paranasal sinus surgery, catheter treatment, and the like. In magnetic navigation surgery, a position of a surgical instrument (non-magnetic) is detected in real time by a magnetic tracking system based on an output of a magnetic sensor attached to the surgical instrument (see Non Patent Document 1).

Patent Document 1 discloses a surgical system that executes optical or magnetic navigation in craniomaxillofacial surgery. Patent Document 2 discloses an optical maxillary alignment system using a mouthpiece type target and a camera.

### Citation List

### Patent Documents

Patent Document 1: US 2017/0000505
Patent Document 2: US 2009/0220122

### Non Patent Document

Non Patent Document 1: "magnetic three-dimensional measurement system AURORA", [online], August 2, 2022, Advanced Systems Co., Ltd., [searched on Aug. 2, 2022], Internet <URL: http://www.asco.jp/02aurora.htm>

### Summary of Invention

### Technical Problem

However, Patent Document 1 discloses a position detection reference module having a complicated shape as a detection reference module used in a magnetic navigation system. There is a problem that the position detection reference module having such a complicated shape interferes with surgery. In addition, the position detection reference module having a shape exposed to the outside detects a position away from an actual position of a bone as a position of the bone. Therefore, there is a problem that even if the bone is accurately moved to the position guided by the navigation system, the position is not an accurate target position, and thus a surgeon cannot align the position of the bone with the accurate target position.

Further, in Patent Document 1, the position detection reference module for detecting a position of a bone fragment to be moved is attached to a cutting guide for cutting and separating a jaw bone from a skull. When such a cutting guide is used, there is a problem that it takes time and effort to align an upper jaw and a lower jaw, and it is difficult to visually dispose a sensor at a target attachment position determined in preoperative planning because there is no noticeable mark in the bone for alignment in many cases. Therefore, it is not preferable that the position detection reference module be attached to the cutting guide.

In Patent Document 2, a maxilla is aligned using a mouthpiece type dental splint (mouthpiece). In such alignment surgery, a maxilla and a mandible are preferably integrated. However, a shape of a target having four light sources for alignment is a shape exposed to the outside from the dental splint, similar to the position detection reference module of Patent Document 1, so that the distance measurement using a camera can be performed. Therefore, the target not only obstructs the surgery, but also the target is away from the position of the dentition, and thus bones may not be accurately aligned with each other. In addition, the target is exposed to the outside from the dental splint, and thus the position of the target as an origin of the alignment may be shifted, and it is difficult to accurately align the bones.

In Patent Document 2, surgeons and caregivers may be crowded around the head of a patient. In such a case, in an optical navigation surgery using a reflector exposed to the outside of the dental splint as in Patent Document 2, optical shielding may occur due to a surgeon or the like. That is, infrared rays reflected by the reflector attached to a surgical instrument may be shielded by the surgeon or the like. When the optical shielding occurs, the infrared rays cannot be used for the distance measurement, and thus a position of the surgical instrument cannot be detected. Such a situation also applies to, for example, a case where a reflector is attached to a bone of a patient who receives surgery.

On the other hand, in the magnetic navigation surgery described in Non Patent Document 1, the position of the surgical instrument or a bone is detected based on the output of the small magnetic sensor attached to the surgical instrument or the bone. Therefore, optical shielding does not occur in the magnetic navigation surgery. For this reason, it has been studied to apply the magnetic navigation surgery using a small magnetic sensor to an oral surgery field.

However, even when the magnetic navigation surgery described in Non Patent Document 1 is applied to orthognathic surgery, a position of a maxilla and a position of a skull may be shifted from each other depending on an attachment position of the magnetic sensor attached to the bone because the bone does not have a mark as in Patent Document 1. As described above, there is a problem that it is difficult to support a surgeon to accurately move and rotate a bone to achieve a target relative position and a target relative posture predetermined by preoperative planning.

As described above, in the bone of the patient on which the orthognathic surgery is performed, there is no mark serving as a guide of a position where a reflector and a sensor such as a magnetic sensor are attached with a screw or the like. Therefore, there is a problem that the surgeon cannot visually grasp an accurate position (target attachment position) where the sensor is required to be attached to the bone of the patient. Further, in the navigation in which the target relative position is presented to the surgeon by a numerical value as in Patent Document 1, there is a problem that the surgeon cannot intuitively grasp whether the bone is accurately moved and rotated such that a relative position and a relative posture of the bone become the target relative position and a target relative posture.

In view of the above circumstances, an object of the invention is to provide a navigation system, a jig, and a program capable of supporting a surgeon in accurately moving and rotating a bone so that a relative position and a relative posture of the bone become a target relative position and a target relative posture.

### Solution to Problem

An aspect of the present invention relates to a navigation system including: an acquisition unit configured to acquire information on a position and a posture of a first sensor detected based on a signal of the first sensor, and information on a target attachment position and a target attachment posture of the first sensor with respect to a position and a posture of a second sensor detected based on a signal of the second sensor, the first sensor being inserted into an attachment portion of a first jig, which has a surface having a shape corresponding to a surface of a first bone, in a first orientation predetermined with respect to the first jig, and the second sensor being inserted into an attachment portion of a second jig, which has a surface having a shape corresponding to a surface of a second bone, in a second orientation predetermined with respect to the second jig; a derivation unit configured to derive a first relative position and a first relative posture of the first sensor with respect to the target attachment position and the target attachment posture based on the target attachment position, the target attachment posture, and the position and the posture of the first sensor; an information generation unit configured to generate a three-dimensional coordinate axis image representing the target attachment position and the target attachment posture and a three-dimensional coordinate axis image representing the first relative position and the first relative posture; and a display unit configured to display the three-dimensional coordinate axis image representing the target attachment position and the target attachment posture and the three-dimensional coordinate axis image representing the first relative position and the first relative posture.

An aspect of the invention relates to a navigation system including: an acquisition unit configured to acquire information on a position and a posture of a first bone detected based on a signal of a first sensor, and information on a position and a posture of a second bone detected based on a signal of a second sensor, the first sensor being inserted into an attachment portion of a first jig, which has a surface having a shape corresponding to a surface of the first bone, in a first orientation predetermined with respect to the first jig, and the second sensor being inserted into an attachment portion of a second jig, which has a surface having a shape corresponding to a surface of the second bone, in a second orientation predetermined with respect to the second jig; a derivation unit configured to derive a relative position and a relative posture of the second bone with respect to the position of the first bone based on the information on the position and the posture of the first bone and the information on the position and the posture of the second bone, and derive a target relative position and a target relative posture of the second bone with respect to the position and the posture of the first bone based on a movement amount predetermined with respect to an initial value of the relative position and a rotation amount predetermined with respect to an initial value of the relative posture; an information generation unit configured to generate a three-dimensional coordinate axis image representing the target relative position and the target relative posture and a three-dimensional coordinate axis image representing the relative position and the relative posture; and a display unit configured to display the three-dimensional coordinate axis image representing the target relative position and the target relative posture and the three-dimensional coordinate axis image representing the relative position and the relative posture.

An aspect of the invention relates to a jig including: an attachment portion configured to allow a sensor to be inserted in a predetermined orientation; and a surface having a shape corresponding to a surface of a bone to be joined.

An aspect of the invention relates to a program for causing a computer to execute steps of: acquiring information on a position and a posture of a first sensor detected based on a signal of the first sensor, and information on a target attachment position and a target attachment posture of the first sensor with respect to a position and a posture of a second sensor detected based on a signal of the second sensor, the first sensor being inserted into an attachment portion of a first jig, which has a surface having a shape corresponding to a surface of a first bone, in a first orientation predetermined with respect to the first jig, and the second sensor being inserted into an attachment portion of a second jig, which has a surface having a shape corresponding to a surface of a second bone, in a second orientation predetermined with respect to the second jig; deriving a first relative position and a first relative posture of the first sensor with respect to the target attachment position and the target attachment posture based on the target attachment position, the target attachment posture, and the position and the posture of the first sensor; generating a three-dimensional coordinate axis image representing the target attachment position and the target attachment posture and a three-dimensional coordinate axis image representing the first relative position and the first relative posture; and displaying the three-dimensional coordinate axis image representing the target attachment position and the target attachment posture and the three-dimensional coordinate axis image representing the first relative position and the first relative posture.

An aspect of the invention relates to a program for causing a computer to execute steps of: acquiring information on a position and a posture of a first bone detected based on a signal of a first sensor, and information on a position and a posture of a second bone detected based on a signal of a second sensor, the first sensor being inserted into an attachment portion of a first jig, which has a surface having a shape corresponding to a surface of the first bone, in a first orientation predetermined with respect to the first jig, and the second sensor being inserted into an attachment portion of a second jig, which has a surface having a shape corresponding to a surface of the second bone, in a second orientation predetermined with respect to the second jig; deriving a relative position and a relative posture of the second bone with respect to the position of the first bone based on the information on the position and the posture of the first bone and the information on the position and the posture of the second bone, and deriving a target relative position and a target relative posture of the second bone with respect to the position and the posture of the first bone based on a movement amount predetermined with respect to an initial value of the relative position and a rotation amount predetermined with respect to an initial value of the relative posture; generating a three-dimensional coordinate axis image representing the target relative position and the target relative posture and a three-dimensional coordinate axis image representing the relative position and the relative posture; and displaying the three-dimensional coordinate axis image representing the target relative position and the target relative posture and the three-dimensional coordinate axis image representing the relative position and the relative posture.

### Advantageous Effects of Invention

According to the invention, it is possible to support a surgeon in accurately moving and rotating a bone so that a relative position and a relative posture of the bone become a target relative position and a target relative posture.

### Brief Description of Drawings

[FIG. 1] A diagram illustrating a configuration example of a navigation system in the first embodiment.
[FIG. 2] A diagram illustrating a skull model and a maxilla model in the first embodiment.
[FIG. 3] A diagram illustrating an example of a position and a posture of the maxilla model before and after movement in the first embodiment.
[FIG. 4] A diagram illustrating a determination example of a movement parameter and a rotation parameter (rotation matrix) in the first embodiment.
[FIG. 5] A diagram illustrating a designation example of an attachment position of a magnetic sensor in the first embodiment.
[FIG. 6] A diagram illustrating an example of a jig joined to a skull in the first embodiment.
[FIG. 7] A diagram illustrating an example of a jig joined to a dentition of a maxilla in the first embodiment.
[FIG. 8] A diagram illustrating an example of a navigation image indicating the attachment position in the first embodiment.
[FIG. 9] A diagram illustrating an example of the navigation image indicating the attachment position in the first embodiment.
[FIG. 10] A diagram illustrating an attachment example of the magnetic sensor in the first embodiment.
[FIG. 11] A diagram illustrating an example of a navigation image indicating a position of a bone and the like in the first embodiment.
[FIG. 12] A diagram illustrating an example of the navigation image indicating the position of the bone and the like in the first embodiment.
[FIG. 13] A flowchart illustrating a first operation example of a navigation device in the first embodiment.
[FIG. 14] A flowchart illustrating a second operation example of the navigation device in the first embodiment.
[FIG. 15] A diagram illustrating a first example of designation of attachment positions of magnetic sensors in the second embodiment.
[FIG. 16] A diagram illustrating an example of cutting a bone in the second embodiment.
[FIG. 17] A diagram illustrating an example of the attachment positions of the magnetic sensors after cutting the bone in the second embodiment.

### Description of Embodiments

Hereinafter, an embodiment of the invention will be described with reference to the drawings.

### (First Embodiment)

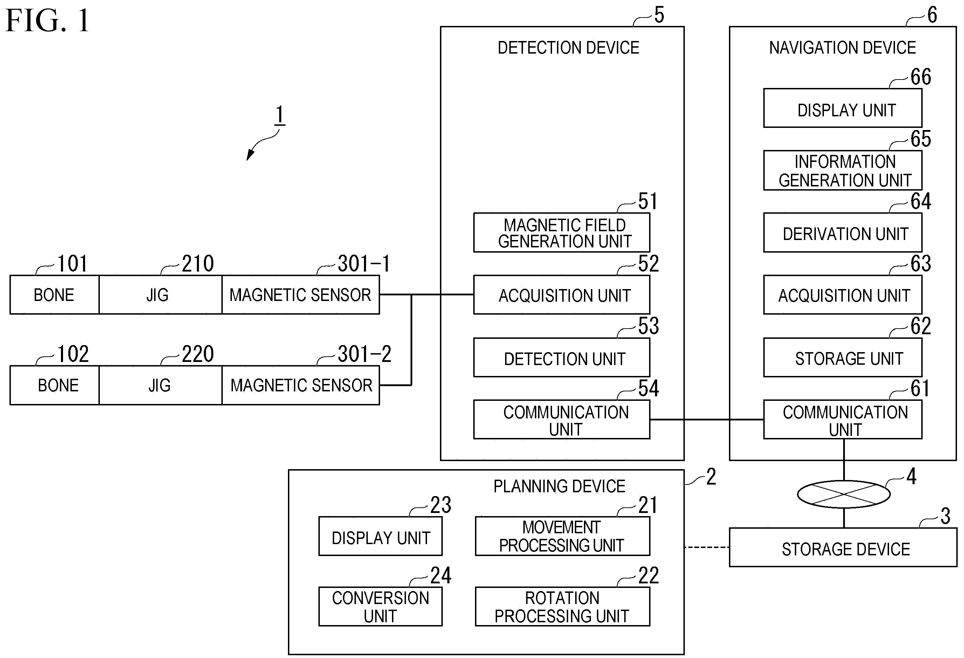

FIG. 1 is a diagram illustrating a configuration example of a navigation system 1 (surgery support system) in the first embodiment. The navigation system 1 is a system that navigates a relative position and a relative posture of a sensor with respect to a target attachment position and a target attachment posture predetermined for a bone of a patient. Accordingly, a surgeon (doctor) can accurately attach the sensor (jig) to the bone of the patient such that a posture of the sensor becomes the target attachment posture at the target attachment position predetermined for the bone of the patient. The target attachment position and the target attachment posture are predetermined by a planner (doctor) in preoperative planning (three-dimensional simulation performed before surgery).

The sensor attached to the bone of the patient may be a light source or a reflector used for optical navigation surgery, or a magnetic sensor used for magnetic navigation surgery. Since optical shielding does not occur, the magnetic sensor is more preferable. Hereinafter, as an example, the magnetic sensor is attached to the bone of the patient by the surgeon.

In addition, the navigation system 1 is a system that navigates a relative position and a relative posture of the bone of the patient with respect to a predetermined target relative position and target relative posture. Accordingly, the surgeon can set a relative position and a relative posture of a second bone with respect to a position and a posture of a first bone to the predetermined target relative position and target relative posture. The target relative position and the target relative posture are predetermined by the planner in the preoperative planning.

The navigation system 1 includes a planning device 2, a storage device 3, a communication line 4, a detection device 5, and a navigation device 6. A jig 210 (first jig) and a magnetic sensor 301-1 (first sensor) are associated with a bone 101 (first bone) of the patient. A jig 220 (second jig) and a magnetic sensor 301-2 (second sensor) are associated with a bone 102 (second bone) of the patient.

In the embodiment of the invention, the jig 220 is preferably a mouthpiece (dental splint). The magnetic sensor 301-2 is attached to the bone 102 by using the jig 220. Since a joining surface of the jig 220 is manufactured to match a shape of a dentition, the surgeon can accurately dispose the magnetic sensor 301-2 at the target attachment position determined on the bone. In the embodiment of the invention, a position of the jig 220 at which the sensor can be accurately disposed at the target attachment position determined on the bone is set as an origin, and the target relative position and the target relative posture are determined with respect to the origin, so that the surgeon can adjust the position of the bone to an accurate position. Note that the jig 220 may not be a mouthpiece as long as the magnetic sensor 301-2 can be accurately disposed at the target attachment position determined on the bone.

Note that the planning device 2 may be provided in the navigation system 1 in the preoperative planning, and may not be provided in the navigation system 1 during surgery.

The planning device 2 (preoperative planning device) includes a movement processing unit 21, a rotation processing unit 22, a display unit 23, and a conversion unit 24. The movement processing unit 21 and the rotation processing unit 22 may be integrated (movement and rotation processing unit). The detection device 5 includes a magnetic field generation unit 51, an acquisition unit 52, a detection unit 53, and a communication unit 54. The navigation device 6 includes a communication unit 61, a storage unit 62, an acquisition unit 63, a derivation unit 64, an information generation unit 65, and a display unit 66.

Some or all of the functional units of the planning device 2 and the navigation device 6 are implemented as software by a processor such as a central processing unit (CPU) executing a program stored in a storage unit including a non-volatile recording medium (non-transitory recording medium) . Further, some of the functional units of the detection device 5 are implemented as software by the processor executing a program stored in a storage unit including a non-volatile recording medium (non-transitory recording medium).

The program may be recorded on a computer-readable recording medium. The computer-readable recording medium is, for example, a non-transitory recording medium such as portable media such as a flexible disk, a magneto optical disk, a read only memory (ROM), or a compact disc read only memory (CD-ROM), and a storage device such as a hard disk built in a computer system or a solid state drive.

Some or all of the functional units of the planning device 2 and the navigation device 6 may be implemented by, for example, hardware including an electronic circuit (or circuitry) using a large scale integrated circuit (LSI), an application specific integrated circuit (ASIC), a programmable logic device (PLD), a field programmable gate array (FPGA), or the like. Further, some of the functional units of the detection device 5 may be implemented by hardware including an electronic circuit using an LSI, an ASIC, a PLD, an FPGA, or the like.

The bone 101 and the bone 102 are bones of the patient. Hereinafter, the bone 101 (first bone) is, for example, a skull (facial bone) of the patient. The bone 102 (second bone) is, for example, a maxilla (Le Fort I bone fragment) separated from the bone 101.

### <Determination of Target Relative Position and Target Relative Posture of Bone 102 Before Surgery>

Prior to the surgery, the preoperative planning is performed by a planner (not illustrated) using the planning device 2. For example, in the preoperative planning performed prior to orthognathic surgery (Le Fort I osteotomy), a skull model (three-dimensional model of the bone 101) is created in a memory of the planning device 2 based on data acquired by performing computed tomography (CT) scanning or the like on the patient.

An image of the skull model is displayed on a screen of the display unit 23 based on the skull model. Then, a maxilla model (three-dimensional model of the bone 102) is separated from the skull model on the screen. Accordingly, images of the separated maxilla model and skull model are displayed on the screen of the display unit 23. A relative position (movement amount) and a relative posture (rotation amount) of the maxilla model with respect to the skull model are examined in response to an operation (for example, a mouse operation) by the planner for a purpose of achieving good occlusion and an aesthetic face for the patient.

Here, the movement processing unit 21 moves the maxilla model on the screen in response to the operation by the planner. The planner checks the movement amount of the maxilla model on the screen to determine the movement amount (X-axis component amount, Y-axis component amount, and Z-axis component amount) with respect to an initial value of a position of the maxilla model. Accordingly, the planner can determine the movement amount with respect to an initial value of an actual position of the bone 102. The movement processing unit 21 records, in the storage device 3, information on the movement amount determined by the planner.

The rotation processing unit 22 may rotate the three-dimensional model of the maxilla model on the screen in response to the operation by the planner. The planner determines the rotation amount with respect to an initial value of the position of the maxilla model by checking the rotation amount (roll axis component amount, pitch axis component amount, and yaw axis component amount) of the maxilla model on the screen. Accordingly, the planner can determine the rotation amount with respect to the initial value of the actual position of the bone 102. The rotation processing unit 22 records, in the storage device 3, information on the rotation amount determined by the planner.

### <Determination of Attachment Position of Sensor Attached to Bone Using Jig Before Surgery>

In the preoperative planning, the planner designates a target attachment position of the magnetic sensor 301-1 (jig 210) in the bone 101 to the conversion unit 24. When the bone 101 is the skull, the target attachment position is, for example, a position determined on a piriform aperture side edge of the skull.

The planner designates the target attachment position of the magnetic sensor 301-1 on the screen of the display unit 23 in a state where the maxilla model is not separated from the skull model on the screen. The planner may designate the target attachment position of the magnetic sensor 301-1 on the screen in a state where the maxilla model is separated from the skull model on the screen of the display unit 23.

The planner joins the model of the jig 220 (mouthpiece) to a dentition of the maxilla model moved and rotated to the target relative position and the target relative posture with respect to the skull model on the screen of the display unit 23. The planner designates the target attachment position of the magnetic sensor 301-1 (jig 210) in the skull model on the screen by, for example, a mouse operation. Accordingly, the planner can input the target attachment position of the magnetic sensor 301-1 in the actual bone 101 to the conversion unit 24. In the embodiment of the invention, the target attachment position of the magnetic sensor 301-1 is expressed with a predetermined position of the jig 220 joined to the dentition of the maxilla model as an origin.

The conversion unit 24 converts a first position (first two-dimensional position information) designated on a surface of the skull model by the planner using the screen (two-dimensional) of the display unit 23 into three-dimensional position information. The three-dimensional position information corresponds to the target attachment position of the magnetic sensor 301-1 (jig 210) in the actual bone 101. The conversion unit 24 records, in the storage device 3, the three-dimensional position information on the target attachment position of the magnetic sensor 301-1.

The conversion unit 24 may convert the second position (second two-dimensional position information) designated on a surface of the maxilla model by the planner using the screen (two-dimensional) of the display unit 23 into three-dimensional position information. The three-dimensional position information corresponds to a target attachment position of the magnetic sensor 301-2 (jig 220) in the actual bone 102. The conversion unit 24 records, in the storage device 3, the three-dimensional position information on the target attachment position of the magnetic sensor 301-2.

The jig 210 and the jig 220 are manufactured by, for example, a three-dimensional printer (3D printer) before the surgery. Note that the conversion unit 24 may determine an orientation (target attachment posture) of a tubular attachment portion included in the jig 210 according to an orientation of the jig 210 attached to the target attachment position in the skull model.

The storage device 3 stores in advance information on a shape of the three-dimensional model of the skull model and information on a shape of the three-dimensional model of the maxilla model. A data format of the three-dimensional models of the skull model and the maxilla model is, for example, digital imaging and communications in medicine (DICOM). Here, a data format of a three-dimensional model of the dentition is, for example, stereolithography (STL). The storage device 3 stores information on the movement amount (movement parameter) and information on the rotation amount (rotation parameter) according to a control signal transmitted from the planning device 2.

### <Outline of Navigation of Attachment Position of Sensor During Surgery>

The detection device 5 generates a magnetic field region in a real space of a predetermined range including the head of the patient and the magnetic sensors 301. The detection device 5 detects (tracks) a position and a posture of each of the magnetic sensors 301 in real time based on an output (signal) of each of the magnetic sensors 301 in the generated magnetic field region.

The surgeon attaches the magnetic sensor 301-1 to the attachment portion of the jig 210. Further, the surgeon attaches the magnetic sensor 301-2 to the attachment portion of the jig 220.

The navigation device 6 presents (guides), to the surgeon in real time, predetermined first information for navigating the position and the posture of the magnetic sensor 301-1 such that the surgeon moves and rotates the jig 210 and the magnetic sensor 301-1 until the position and the posture of the magnetic sensor 301-1 match the target attachment position and the target attachment posture of the magnetic sensor 301-1 predetermined on the bone 101 of the patient with a position of the magnetic sensor 301-2 inserted into the jig 220 as an origin.

A jig is always attached to the piriform aperture side edge and the mandible, but there is no noticeable mark on the piriform aperture side edge and the mandible. Therefore, it has been difficult to visually dispose the jigs on the piriform aperture side edge and the mandible according to the preoperative planning. In the embodiment of the invention, a position of an attachment portion 221 provided in the jig 220 capable of accurately attaching the magnetic sensor 301-2 to the position (origin) determined in the mandible is set as the origin. Accordingly, it is possible to present (guide) the surgeon with an accurate attachment position of the jig 210 on the piriform aperture side edge.

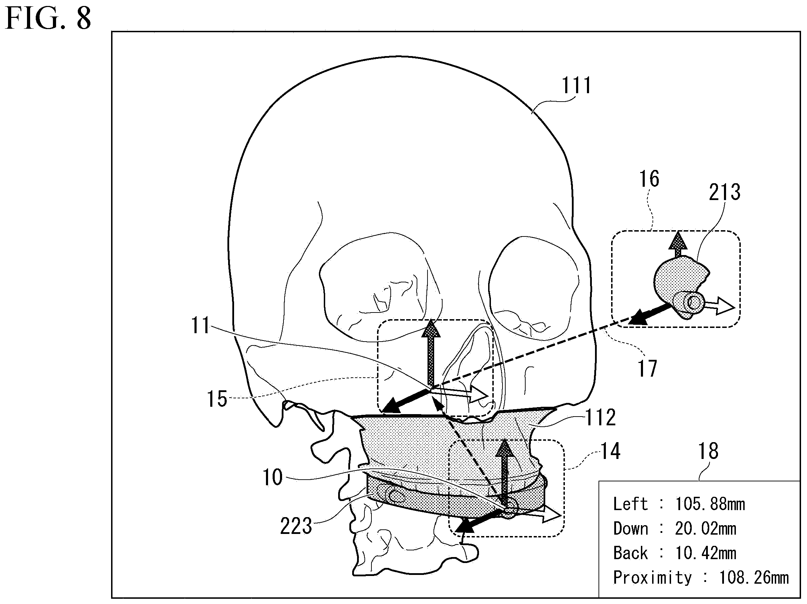

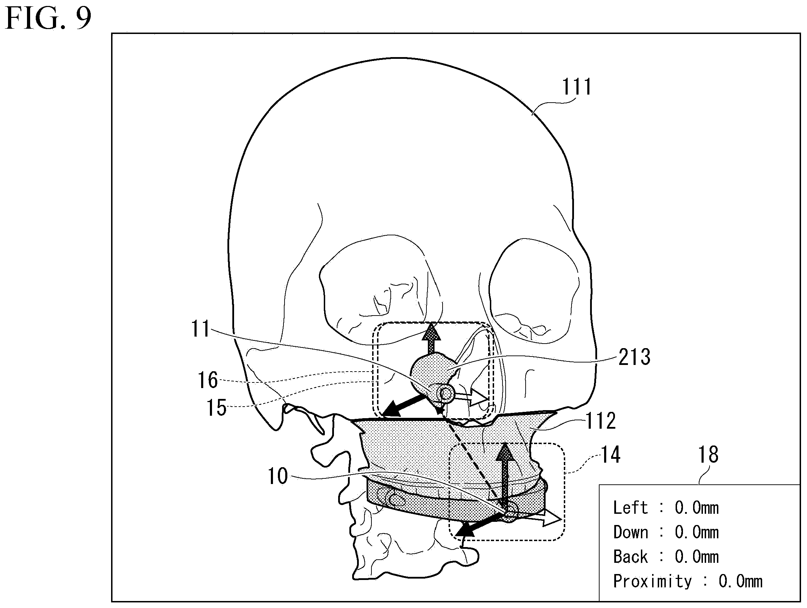

The predetermined first information includes, for example, at least one of an image of the three-dimensional model (skull model) of the bone 101 whose shape is measured in advance, an image of the three-dimensional model (maxilla model) of the bone 102 whose shape is measured in advance, a three-dimensional coordinate axis image representing the relative position and the relative posture of each of the magnetic sensors 301 (each of the jigs), and a three-dimensional coordinate axis image representing the target attachment position and the target attachment posture. Details of the first information will be described later with reference to FIGS. 8 and 9.

By presenting such first information to the surgeon in real time, the navigation system 1 supports the surgeon in accurately attaching the jig 210 (magnetic sensor 301-1) such that the relative posture of the magnetic sensor 301-1 becomes the target attachment posture at the target attachment position predetermined on the bone 101.

### <Details of Navigation of Attachment Position of Sensor During Surgery>

The jig 210 is a jig for fixing the magnetic sensor 301-1 to the bone 101. The jig 210 is temporarily fixed to the bone 101 by the surgeon. The jig 220 is a jig for fixing the magnetic sensor 301-2 to the bone 102. The jig 220 is temporarily fixed to the dentition of the bone 102 by the surgeon.

The magnetic sensor 301-1 is disposed in the vicinity of the bone 101 by using the jig 210. The vicinity of the bone is a range from about 0.1 mm to about 10 mm from the bone, for example. In the embodiment of the invention, the magnetic sensor 301-1 is inserted into a hole of an attachment portion 211 provided in the jig 210. Since the magnetic sensor 301-1 is disposed in the vicinity of the bone 101, the bone 101 can be accurately aligned.

The magnetic sensor 301 is a sensor that detects magnetism. The magnetic sensor 301 detects magnetism at a position of the own sensor in a magnetic field generated by the magnetic field generation unit 51 at a predetermined period (for example, 40 Hz) using a magnetic detection device such as a coil. The magnetic sensor 301 outputs an electric signal corresponding to the detected magnetism to the acquisition unit 52.

The magnetic sensor 301-1 is temporarily attached to the target attachment position predetermined on a surface of the bone 101 (skull) at by using the jig 210. That is, the magnetic sensor 301-1 is temporarily attached to the target attachment position predetermined on the surface of the bone 101 by being inserted into the attachment portion of the jig 210 temporarily fixed to the bone 101. Therefore, the position and the posture detected based on the output (signal) of the magnetic sensor 301-1 attached to the bone 101 represent a position and a posture of the bone 101.

The magnetic sensor 301-2 is temporarily attached to the target attachment position predetermined on a surface of the bone 102 using the jig 220 joined to the dentition of the bone 102. Similarly to the insertion of the magnetic sensor 301-1 into the hole of the attachment portion 211 provided in the jig 210, the magnetic sensor 301-2 is inserted into a hole of the attachment portion 221 provided in the jig 220, so that the magnetic sensor 301-2 is attached to the vicinity of the bone 102 by using the jig 220. That is, the magnetic sensor 301-2 is temporarily attached to the target attachment position predetermined on the surface of the bone 102 by being inserted into the attachment portion of the jig 220 temporarily fixed to the dentition of the bone 102. Therefore, the position and the posture detected based on the output of the magnetic sensor 301-2 attached to the bone 102 represent a position and a posture of the bone 102.

The magnetic field generation unit 51 generates a magnetic field region in a real space of a predetermined range including the bone 101, the bone 102, and the magnetic sensors 301 (for example, a real space of a range including the head of the patient). The acquisition unit 52 acquires an output (electric signal corresponding to magnetism) of the magnetic sensor 301. The detection unit 53 detects the position and the posture of the magnetic sensor 301 at a predetermined period (for example, 40 Hz) based on the output of the magnetic sensor 301. A degree of freedom of the detected position includes three degrees of freedom (X-axis, Y-axis, and Z-axis). The degree of freedom of the detected posture (inclination) includes three degrees of freedom (roll axis, pitch axis, and yaw axis). The communication unit 54 transmits information on the position and the posture (six degrees of freedom in total) of the magnetic sensor 301 to the communication unit 61 at a predetermined period (for example, 40 Hz).

The communication unit 61 acquires the information on the shape of the three-dimensional model of the bone 101 and the information on the shape of the three-dimensional model of the bone 102 from the storage device 3. The communication unit 61 acquires information on the target attachment position of the magnetic sensor 301-1 (jig 210) from the storage device 3. The communication unit 61 may acquire information on the target attachment posture of the magnetic sensor 301-1 (jig 210) from the storage device 3. The communication unit 61 acquires information on the position and the posture of each of the magnetic sensors 301 from the communication unit 54 at a predetermined period.

The storage unit 62 stores the information on the target attachment position of the magnetic sensor 301-1 (jig 210). The storage unit 62 stores the information on the target attachment posture of the magnetic sensor 301-1 (jig 210). The storage unit 62 (buffer memory) temporarily stores time series information on the position and the posture of each of the magnetic sensors 301. The storage unit 62 temporarily stores time series information on the position and the posture of the bone 101 and time series information on the position and the posture of the bone 102. The storage unit 62 may store in advance a computer program executed by the derivation unit 64 and the information generation unit 65.

The acquisition unit 63 acquires the information on the shape of the bone 101 and the information on the shape of the bone 102 from the storage device 3. The acquisition unit 63 acquires information on the target attachment position and the target attachment posture of the magnetic sensor 301-1 from the communication unit 61 or the storage unit 62. The information on the position and the posture of the magnetic sensor 301-1 (jig 210) and the information on the position and the posture of the magnetic sensor 301-2 (jig 220) are acquired from the communication unit 61 or the storage unit 62 at a predetermined period.

The derivation unit 64 derives the target attachment position and the target attachment posture predetermined on the surface of the bone 101 (skull) in real time based on the information on the target attachment position and the target attachment posture designated by the planner, with the position of the magnetic sensor 301-2 attached to the jig 220 joined to the dentition as the origin. The derivation unit 64 derives the relative position and the relative posture of the magnetic sensor 301-1 (jig 210) with respect to the target attachment position and the target attachment posture in real time based on the acquired information on the position and the posture of the magnetic sensor 301-1.

The information generation unit 65 generates a navigation image at a predetermined frame rate (for example, 40 fps) by using a predetermined image processing engine (three-dimensional computer graphics drawing engine). The navigation image includes, for example, an image representing an appearance (shape) of the bone generated in the preoperative planning, predetermined character information, and a three-dimensional coordinate axis image.

The information generation unit 65 generates an image representing an appearance of the bone 101 according to the position and the posture of the bone 101 (image of the skull model) and an image representing an appearance of the bone 102 according to the relative position and the relative posture of the bone 102 (image of the maxilla model).

When each of the magnetic sensors 301 (each of the jigs) is attached to each of the bones during the surgery, the information generation unit 65 generates a three-dimensional coordinate axis image representing a current position and posture of the magnetic sensor 301 (jig). In addition, the information generation unit 65 generates a three-dimensional coordinate axis image representing the target attachment position and the target attachment posture of the magnetic sensor 301-1.

The display unit 66 displays the navigation image generated by the information generation unit 65 at a predetermined frame rate (for example, 40 fps). For example, the display unit 66 displays the three-dimensional coordinate axis image representing the current position and posture of the magnetic sensor 301-1 (jig 210) and the three-dimensional coordinate axis image representing the target attachment position and the target attachment posture of the magnetic sensor 301-1 (jig 210).

The surgeon joins the jig 220, into which the magnetic sensor 301-2 is inserted, to the dentition of the bone 102 of the patient. Further, the surgeon accurately attaches the jig 210, into which the magnetic sensor 301-1 is inserted, to the target attachment position predetermined on the bone 101 of the patient with a screw (metal screw) or the like while checking the navigation image. Here, the surgeon accurately attaches the magnetic sensor 301-1 to the bone 101 while checking the navigation image such that the relative posture of the magnetic sensor 301-1 with respect to the posture of the magnetic sensor 301-2 becomes the target attachment posture.

### <Outline of Navigation of Relative Movement and Relative Rotation of Bone During Surgery>

The surgeon separates the bone 102 (maxilla) from the bone 101 (skull). Here, the surgeon may temporarily remove the jig from the bone and then separate the bone 102 (maxilla) from the bone 101 (skull).

The detection device 5 generates a magnetic field region in a real space of a predetermined range including the head of the patient and the magnetic sensors 301. The detection device 5 detects (tracks) a position and a posture of each of the magnetic sensors 301 in real time based on an output (signal) of the magnetic sensor 301 in the generated magnetic field region.

The navigation device 6 presents (guides), to the surgeon in real time, predetermined second information for navigating movement and rotation of the bone 102 by the surgeon, so that the surgeon moves and rotates the bone 102 until the relative position and the relative posture of the bone 102 with respect to the position and the posture of the bone 101 match the target relative position and the target relative posture.

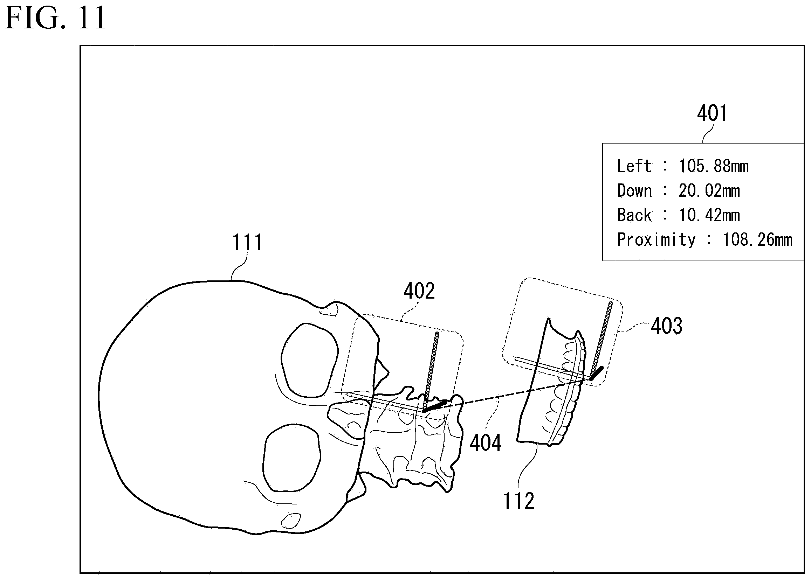

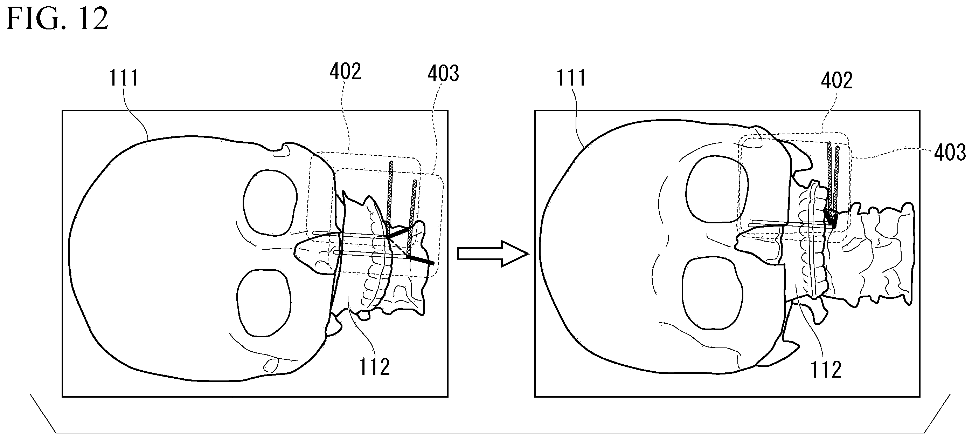

The predetermined second information includes, for example, at least one of the image of the three-dimensional model (skull model) of the bone 101 whose shape is measured in advance, the image of the three-dimensional model (maxilla model) of the bone 102 whose shape is measured in advance, a three-dimensional coordinate axis image representing the position and the posture of the bone 101, a three-dimensional coordinate axis image representing the relative position and the relative posture of the bone 102 with respect to the position and the posture of the bone 101, and a three-dimensional coordinate axis image representing the target relative position and the target relative posture of the bone 102. Details of the second information will be described later with reference to FIGS. 11 and 12.

By presenting such second information to the surgeon in real time, the navigation system 1 supports the surgeon in accurately moving and rotating (tilting) the bone 102 such that the position and the posture of the bone 102 become the target relative position and the target relative posture.

### <Details of Navigation of Movement and Rotation of Bone During Surgery>

The communication unit 61 acquires the information on the shape of the three-dimensional model (skull model) of the bone 101 and the information on the shape of the three-dimensional model (maxilla model) of the bone 102 from the storage device 3. The communication unit 61 acquires information on a predetermined movement amount and information on a predetermined rotation amount from the storage device 3. The communication unit 61 acquires the information on the position and the posture of the bone 101 and the information on the position and the posture of the bone 102 from the communication unit 54 at a predetermined period.

The storage unit 62 stores the information on the predetermined movement amount and the information on the predetermined rotation amount. The storage unit 62 (buffer memory) temporarily stores the time series information on the position and the posture of the bone 101 and the time series information on the position and the posture of the bone 102. The storage unit 62 may store in advance a computer program executed by the derivation unit 64 and the information generation unit 65.

The acquisition unit 63 acquires the information on the shape of the three-dimensional model (skull model) of the bone 101 and the information on the shape of the three-dimensional model (maxilla model) of the bone 102 from the storage device 3. The acquisition unit 63 acquires the information on the predetermined movement amount and the information on the predetermined rotation amount from the communication unit 61 or the storage unit 62. The acquisition unit 63 acquires the information on the position and the posture of the magnetic sensor 301-1 (jig 210) and the information on the position and the posture of the magnetic sensor 301-2 (jig 220) from the communication unit 61 or the storage unit 62 at a predetermined period.

When the magnetic sensor 301-1 (jig 210) is attached to the bone 101, the acquisition unit 63 acquires the information on the position and the posture of the magnetic sensor 301-1 from the communication unit 61 or the storage unit 62 at a predetermined period as the information on the position and the posture of the bone 101. Similarly, when the magnetic sensor 301-2 (jig 220) is attached to the bone 102, the acquisition unit 63 acquires the information on the position and the posture of the magnetic sensor 301-2 from the communication unit 61 or the storage unit 62 at a predetermined period as the information on the position and the posture of the bone 102.

The derivation unit 64 derives the relative position and the relative posture of the magnetic sensor 301-2 with respect to the position and the posture of the magnetic sensor 301-1 in real time based on the information on the position and the posture of the magnetic sensor 301-1 and the information on the position and the posture of the magnetic sensor 301-2. That is, the derivation unit 64 derives the relative position and the relative posture of the bone 102 with respect to the position and the posture of the bone 101 in real time based on the information on the position and the posture of the bone 101 and the information on the position and the posture of the bone 102.

The derivation unit 64 derives the target relative position and the target relative posture of the bone 102 with respect to the position and the posture of the bone 101 in real time based on the movement amount and the rotation amount predetermined with respect to initial values of the relative position and the relative posture of the bone 102 in the preoperative planning and the relative position and the relative posture of the bone 102. Here, the position and the posture obtained by moving and rotating the relative position and the relative posture of the bone 102 by the predetermined movement amount and rotation amount are the target relative position and the target relative posture of the bone 102.

The information generation unit 65 generates a navigation image at a predetermined frame rate by using a predetermined image processing engine. The navigation image includes, for example, an image representing an appearance (shape) of the bone generated in the preoperative planning, predetermined character information, and a three-dimensional coordinate axis image.

The information generation unit 65 generates an image representing an appearance of the bone 101 according to the position and the posture of the bone 101 (image of the skull model) and an image representing an appearance of the bone 102 according to the relative position and the relative posture of the bone 102 (image of the maxilla model). For example, when the bone 101 to which the magnetic sensor 301-1 is attached is oriented sideways, the information generation unit 65 generates an image representing the appearance of the bone 101 oriented sideways (image of the skull model oriented sideways). For example, when the bone 102 to which the magnetic sensor 301-2 is attached moves, the information generation unit 65 moves the image representing the appearance of the bone 102 on the screen in response to the movement of the bone 102.

The information generation unit 65 generates, for example, the three-dimensional coordinate axis image representing the target relative position and the target relative posture of the bone 102 based on the target relative position and the target relative posture of the bone 102 with respect to the position and the posture of the bone 101. For example, when the bone 101 to which the magnetic sensor 301-1 is attached is oriented sideways, the target relative position and the target relative posture of the bone 102 are also oriented sideways, and thus the information generation unit 65 generates the three-dimensional coordinate axis image representing the target relative position and the target relative posture of the bone 102 in a sideways orientation.

The information generation unit 65 generates, for example, the three-dimensional coordinate axis image representing the relative position and the relative posture of the bone 102 based on the relative position and the relative posture of the bone 102 with respect to the position and the posture of the bone 101. Also, the information generation unit 65 may generate an image of a line connecting the relative position and the target relative position of the bone 102. For example, when the bone 102 to which the magnetic sensor 301-2 is attached moves, the information generation unit 65 moves the three-dimensional coordinate axis image representing the relative position and the relative posture of the bone 102 on the screen in response to the movement of the bone 102.

The display unit 66 displays the navigation image generated by the information generation unit 65 at a predetermined frame rate (for example, 40 fps).

Next, the preoperative planning before the surgery will be described in more detail.

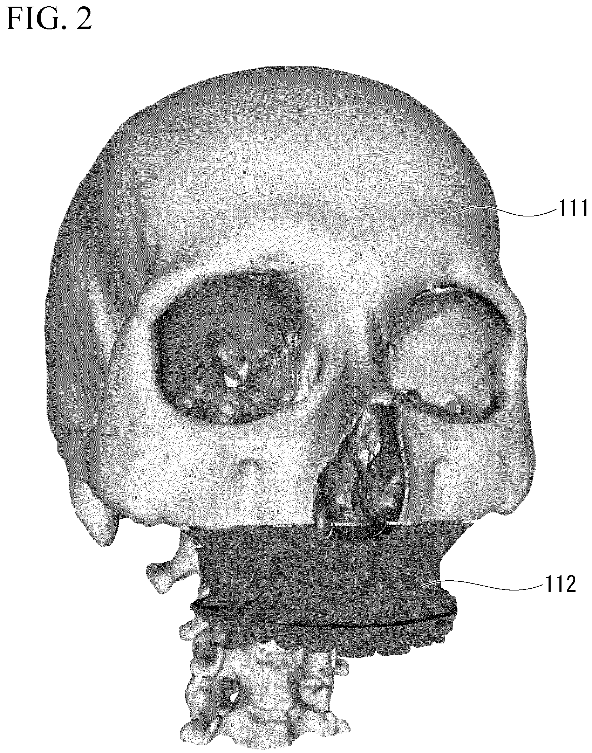

FIG. 2 is a diagram illustrating a skull model 111 and a maxilla model 112 in the first embodiment. The skull model 111 corresponds to the bone 101 in the real space. The maxilla model 112 corresponds to the bone 102 in the real space.

Information (DICOM data) on shapes of the skull model 111 and the maxilla model 112 is obtained, for example, as a result of a CT scan performed on the head of the patient before the surgery. Note that data (STL data) obtained as a result of optical digital scanning performed on the dentition of the patient before the surgery may be used as dentition data of the maxilla model 112.

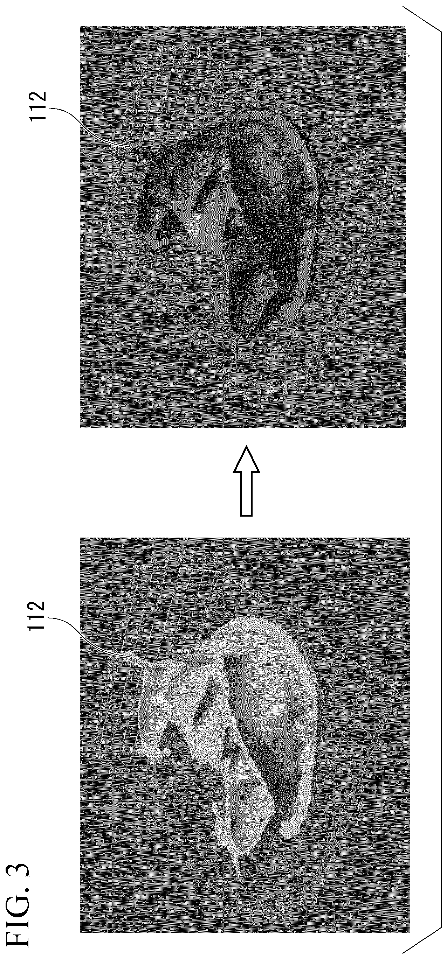

FIG. 3 is a diagram illustrating an example of a position and a posture of the maxilla model 112 before and after movement in the first embodiment. In the preoperative planning, the movement processing unit 21 moves the maxilla model 112 on the screen from an initial value of the position of the maxilla model 112 in response to a mouse operation or the like by the planner (doctor). The rotation processing unit 22 rotates the maxilla model 112 on the screen from an initial value of the posture of the maxilla model 112 in response to the mouse operation or the like by the planner. The diagram on the left side illustrated in FIG. 3 represents a position (initial value) and a posture (initial value) of the skull model 111 before the movement and before the rotation. The diagram on the right side illustrated in FIG. 3 represents a position and a posture of the skull model 111 after the movement and after the rotation.

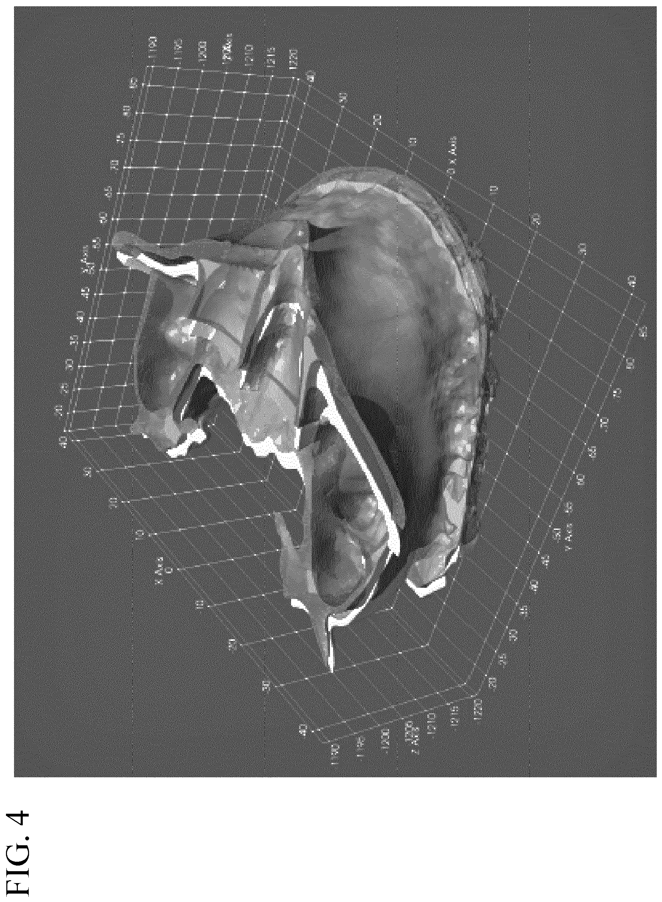

FIG. 4 is a diagram illustrating a determination example of a movement parameter and a rotation parameter (rotation matrix) in the first embodiment. In the preoperative planning, the planner determines the relative position and the relative posture of the bone 102 after the surgery by moving and rotating the maxilla model 112 from the initial values of the position and the posture of the maxilla model 112 on the screen. At least one of the movement processing unit 21 and the rotation processing unit 22 determines at least one of the movement amount and the rotation amount by using a predetermined algorithm for alignment. The predetermined algorithm for alignment is, for example, an iterative closest point (ICP) algorithm. The movement processing unit 21 records information (movement parameter) on the determined movement amount in the storage device 3. The rotation processing unit 22 records information (rotation parameter) on the determined rotation amount in the storage device 3.

Next, designation of the attachment position of the magnetic sensor (jig) before the surgery will be described.

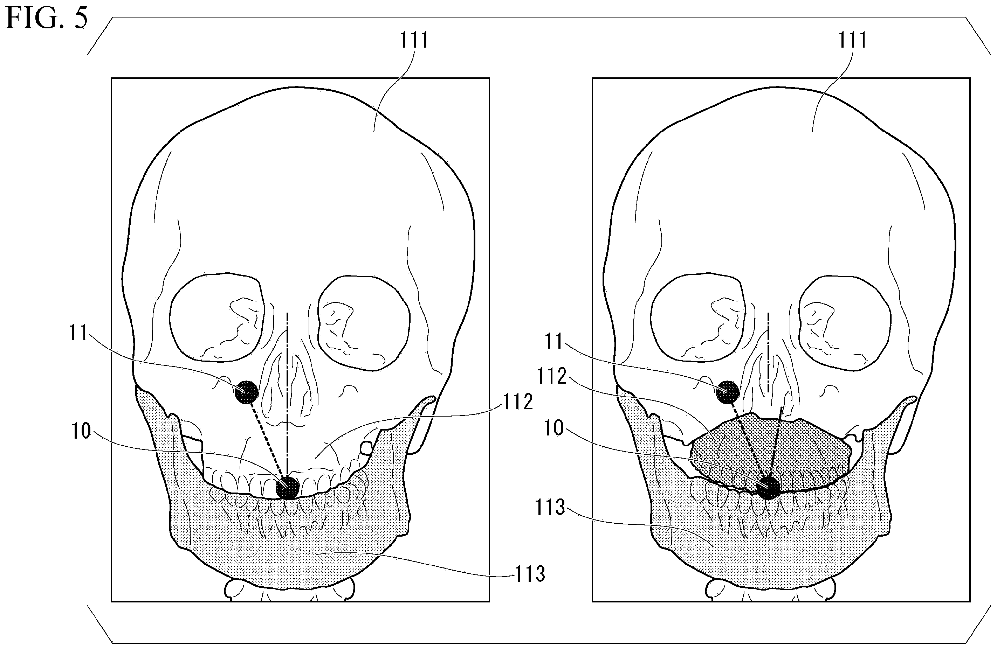

FIG. 5 is a diagram illustrating a designation example of the attachment position (target attachment position) of the magnetic sensor 301 in the first embodiment. On the left side in FIG. 5, the attachment position of the magnetic sensor 301 is illustrated on the screen in a state before the maxilla model 112 is separated from the skull model 111. On the other hand, on the right side in FIG. 5, the attachment position of the magnetic sensor 301 is illustrated on the screen in a state where the maxilla model 112 is separated from the skull model 111 and the maxilla model 112 is rejoined to the skull model 111.

In FIG. 5, the jig 220 illustrated in FIG. 1 is sandwiched between and joined to a dentition of the maxilla model 112 and a dentition of the mandible model 113. An attachment position 10 represents an attachment position of the magnetic sensor 301-2 (jig 220) actually attached to the bone 102. An attachment position 11 represents a target attachment position of the magnetic sensor 301-1 actually attached to the bone 101. The attachment position 11 is expressed with the attachment position 10 as the origin.

In the preoperative planning, the planner designates the attachment position 10 as the attachment position (origin) of the magnetic sensor 301-2 to the bone 102 by marking a surface of the dentition of the maxilla model 112 by, for example, a mouse operation. The attachment position (origin) of the magnetic sensor 301-2 is the position of the attachment portion of the magnetic sensor 301-2 in the jig 220. Further, the planner determines the attachment position 11 as the attachment position (target attachment position) of the magnetic sensor 301-1 to the bone 101 by, for example, marking a surface (the piriform aperture edge of the skull) of the skull model 111.

As illustrated on the left side in FIG. 5, the planner designates the attachment position 10 and the attachment position 11 on the screen, for example, in a state where the maxilla model 112 is not separated from the skull model 111.

As illustrated on the right side in FIG. 5, for example, the planner may designate the attachment position 10 and the attachment position 11 on the screen in the state where the maxilla model 112 is separated from the skull model 111 and the maxilla model 112 is rejoined to the skull model 111. Here, the conversion unit 24 may correct the attachment position 11 in the state where the maxilla model 112 is rejoined to the skull model 111 (right side in FIG. 5), to the attachment position 11 in the state where the maxilla model 112 is not separated from the skull model 111 (left side in FIG. 5). Accordingly, even when the attachment position 11 is designated in the state where the maxilla model 112 is rejoined to the skull model 111 in the preoperative planning, the surgeon can accurately attach the magnetic sensor 301-1 (jig 210) to the attachment position 11 before separating the bone 102 from the bone 101.

Next, jig fabrication (jig shaping) before the surgery will be described.

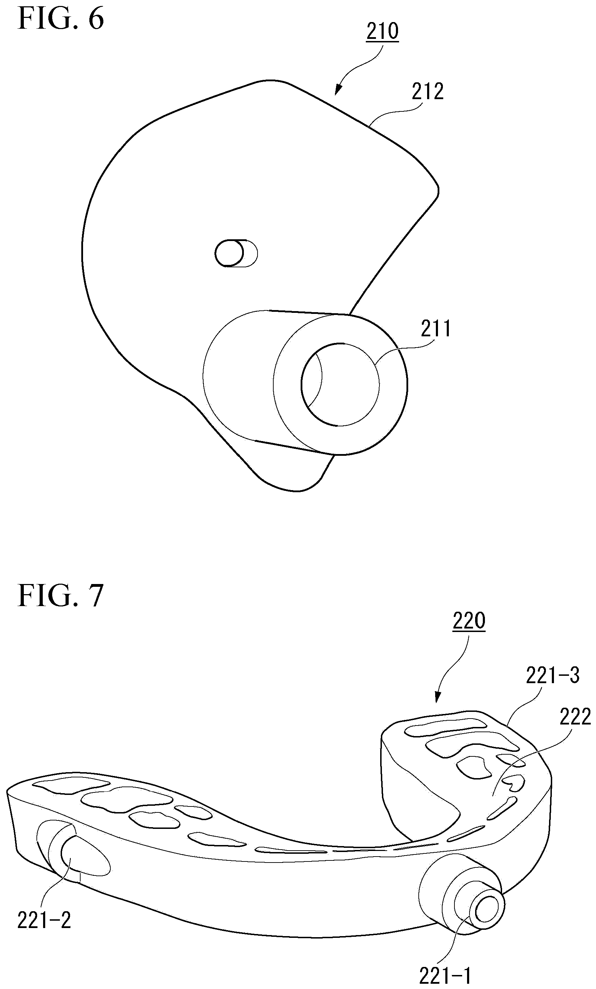

FIG. 6 is a diagram illustrating an example of a jig joined to the skull (bone 101) in the first embodiment. The jig 210 is created using a computer-aided design/computer-aided manufacturing (CAD/CAM) method. That is, design data of a shape of the jig 210 is designed based on the DICOM data of the skull model 111. In addition, the jig 210 is manufactured by, for example, a three-dimensional printer (3D printer) based on the design data (for example, STL data) of the shape of the jig 210.

The jig 210 is made of a non-magnetic material, for example, resin. The jig 210 has one or more attachment portions 211 for attaching the magnetic sensor 301-1, and a joining surface having a shape corresponding to a surface shape of the bone 101 (piriform aperture edge of the skull) to be joined.

The attachment portion 211 has, for example, a tubular shape. A distal end portion (magnetic detection device) of the magnetic sensor 301-1 can be inserted into the attachment portion 211. Since the magnetic sensor 301-1 is disposed in the vicinity of the bone 101 by inserting the magnetic sensor 301-1 into the attachment portion 211, the position of the magnetic sensor 301-1 accurately represents the position of the bone 101. Accordingly, the relative position of the bone 102 can be aligned with the target relative position with high accuracy.

When the jig 210 into which the magnetic sensor 301-1 is inserted is attached to the bone 101, an orientation of each of the attachment portions is designed based on the target attachment posture of the jig 210 such that the orientation of the magnetic sensor 301-1 inserted into the attachment portion 211 (first orientation predetermined with respect to the jig 210) and the orientation of the magnetic sensor 301-2 inserted into the attachment portion 221 of the jig 220 (second orientation predetermined with respect to the jig 220) are the same. In addition, the shape of the joining surface of the jig 210 is designed to match the surface shape of the bone 101 at the target attachment position of the jig 210.

FIG. 7 is a diagram illustrating an example of the jig 220 (mouthpiece) (dental splint) to be joined to the dentition of the maxilla (bone 102) in the first embodiment. In the orthognathic surgery, the dentition of the patient is corrected before the surgery so that the dentition of the patient is aligned optimally after the surgery. The jig 220 is a computer-aided design/computer-aided manufacturing sprint (CAD/CAM sprint) having a joining surface of a shape corresponding to the surface shape of the dentition so that the jig 220 is accurately joined to the corrected dentition. That is, design data of a shape of the jig 220 is designed based on the STL data of the maxilla model 112. Further, the jig 220 is manufactured by, for example, a three-dimensional printer based on the design data of the shape of the jig 220. Since the jig 220 is joined to the dentition of the patient, the jig 220 is accurately fixed to the maxilla (bone 102) of the patient.

The jig 220 is made of a non-magnetic material, for example, resin. The jig 220 includes one or more attachment portions 221 for attaching the magnetic sensor 301-2, and a joining surface 222 having a shape corresponding to a surface shape of the bone 102 (dentition in the maxilla). The attachment portion 221 has, for example, a tubular shape. A distal end portion (magnetic detection device) of the magnetic sensor 301-2 can be inserted into the attachment portion 221. Since the magnetic sensor 301-2 is disposed in the vicinity of the bone 102 by inserting the magnetic sensor 301-2 into the attachment portion 221, the position of the magnetic sensor 301-2 accurately represents the position of the bone 102.

Among an attachment portion 221-1, an attachment portion 221-2, and an attachment portion 221-3, a position of the attachment portion 221-1 is, for example, a position passing through or near a median line of the patient when the joining surface 222 is joined to the dentition of the bone 102. The magnetic sensor 301-2 may be attached to any one of the attachment portion 221-1, the attachment portion 221-2, and the attachment portion 221-3.

When the mouthpiece type jig 220 is joined to the bone 102 and the mandible (not illustrated), the bone 102 and the mandible (not illustrated) are integrated. That is, the position of the mandible of the patient follows the position of the bone 102 (maxilla) moved by the surgeon. Since the maxilla and the mandible do not shift during the movement, navigation is executed more easily.

Next, navigation of the attachment position of the magnetic sensor 301 (jig) during the surgery will be described in more detail.

FIG. 8 is a diagram illustrating an example of a navigation image indicating the attachment position in the first embodiment. During the surgery, the display unit 66 displays a navigation image indicating the relative position and the relative posture of the magnetic sensor 301-1 with respect to the target attachment position and the target attachment posture of the magnetic sensor 301-1 (jig 210) in real time at a predetermined frame rate. FIG. 8 illustrates an example of a navigation image at a first timing during the surgery.

The navigation image illustrated in FIG. 8 includes an image of the skull model 111, an image of the maxilla model 112, a jig image 213, a jig image 223, a coordinate axis image 14 disposed at the attachment position 10, a coordinate axis image 15 disposed at the attachment position 11, a coordinate axis image 16, a line image 17, and a display region 18.

The jig image 213 is an image of a three-dimensional model of the jig 210. The jig image 223 is an image of a three-dimensional model of the jig 220. The attachment position 10 is an attachment position (origin) of the magnetic sensor 301-2. The attachment position 11 is a target attachment position of the magnetic sensor 301-1 (jig 210).

The coordinate axis image 14 is represented by three line segments (X-axis, Y-axis, and Z-axis) orthogonal to each other. An origin of the coordinate axis image 14 represents the position (attachment position 10) of the magnetic sensor 301-2 attached to the jig 220. A posture of the coordinate axis image 14 represents the posture of the magnetic sensor 301-2 attached to the attachment portion 221 of the jig 220.

The coordinate axis image 15 is represented by three line segments (X-axis, Y-axis, and Z-axis) orthogonal to each other. An origin of the coordinate axis image 15 represents the target attachment position (attachment position 11) of the magnetic sensor 301-1. The posture of the coordinate axis image 15 represents the target attachment posture of the magnetic sensor 301-1.

The coordinate axis image 16 is represented by three line segments (X-axis, Y-axis, and Z-axis) orthogonal to each other. An origin of the coordinate axis image 16 represents the relative position of the magnetic sensor 301-1 attached to the jig 210 with respect to the attachment position 11 (target attachment position). A posture of the coordinate axis image 16 represents the posture of the magnetic sensor 301-1 attached to the attachment portion 211 of the jig 210.

The line image 17 is a line connecting the target attachment position and the relative position of the magnetic sensor 301-1. A length of the line image 17 represents a difference (distance) between the target attachment position and the relative position of the magnetic sensor 301-1.

A color of the X-axis of the coordinate axis image 14, a color of the X-axis of the coordinate axis image 15, and a color of the X-axis of the coordinate axis image 16 are the same color (for example, red). A color of the Y-axis of the coordinate axis image 14, a color of the Y-axis of the coordinate axis image 15, and a color of the Y-axis of the coordinate axis image 16 are the same color (for example, blue). A color of the Z-axis of the coordinate axis image 14, a color of the Z-axis of the coordinate axis image 15, and a color of the Z-axis of the coordinate axis image 16 are the same color (for example, green). A color of the line image 17 connecting the origin (target attachment position) of the coordinate axis image 15 and the origin (relative position of the magnetic sensor 301-1 at a current timing) of the coordinate axis image 16 is purple, for example.

The display region 18 is a region in which at least one of numerical value information and character information is displayed. The numerical value information displayed in the display region 18 represents a difference between the target attachment position and the relative position of the magnetic sensor 301-1. In addition, the character information displayed in the display region 18 represents a movement direction in which the difference between the target attachment position and the relative position of the magnetic sensor 301-1 is reduced.

The surgeon attaches the magnetic sensor 301-1 to the attachment portion 211 of the jig 210. The surgeon attaches the magnetic sensor 301-2 to the attachment portion 221-1 of the jig 220. The surgeon joins the jig 220 to the bone 102. The surgeon moves and rotates the actual jig 210 such that the coordinate axis image 15 and the coordinate axis image 16 overlap on the screen of the display unit 66 while checking the navigation image displayed in real time on the display unit 66.

FIG. 9 is a diagram illustrating an example of a navigation image indicating the attachment position in the first embodiment. FIG. 9 illustrates an example of a navigation image at a second timing after the first timing. At the second timing, as a result of the jig 210 being moved and rotated by the surgeon, the coordinate axis image 15 and the coordinate axis image 16 substantially overlap each other. In this case, the relative position and the target attachment position of the jig 210 substantially match each other. Also, the relative posture and the target attachment posture of the jig 210 substantially match each other.

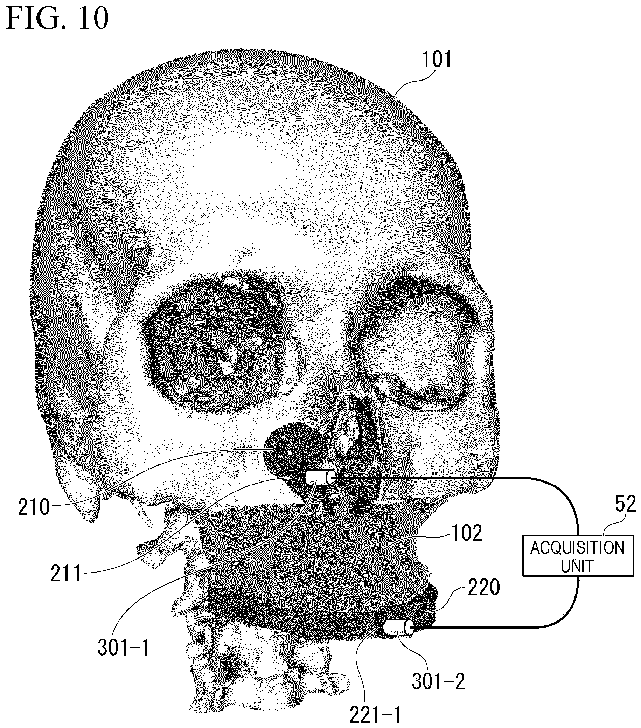

FIG. 10 is a diagram illustrating an attachment example of the magnetic sensor 301 in the first embodiment. In FIG. 10, the jig 210 is temporarily fixed to the bone 101 using a small screw or the like in a state where a joining surface 212 of the jig 210 is joined to the bone 101 (piriform aperture edge of the skull). In addition, the jig 220 is temporarily fixed to the bone 102 in a state where the joining surface 222 of the jig 220 is joined to the dentition of the bone 102.

Here, the orientation (predetermined orientation) of the attachment portion 211 of the jig 210 joined to the bone 101 is the same as the orientation (predetermined orientation) of each of the attachment portions 221 of the jig 220 joined to the bone 102. Accordingly, calibration of the direction of each of the magnetic sensors 301 (initial value of the posture of each of the bones) can be omitted.

Next, the navigation of the position of the bone during the surgery will be described in more detail.

FIG. 11 is a diagram illustrating an example of a navigation image illustrating relative positions of bones and the like in the first embodiment. The display unit 66 displays the navigation image generated by the information generation unit 65 at a predetermined frame rate (for example, 40 fps). In the navigation image, the shape of the bone 101 is represented using the image of the skull model 111. The shape of the bone 102 is represented by using the image of the maxilla model 112.

The navigation image illustrated in FIG. 11 includes the image of the skull model 111, the image of the maxilla model 112, a display region 401, a coordinate axis image 402, a coordinate axis image 403, and a line image 404. The display region 401 is a region in which at least one of numerical value information and character information is displayed. The numerical value information displayed in the display region 401 represents a difference between the target relative position of the bone 102 and the relative position of the bone 102. In addition, the character information displayed in the display region 401 represents a movement direction in which the difference between the target relative position of the bone 102 and the relative position of the bone 102 is reduced.

The coordinate axis image 402 is represented by three line segments (X-axis, Y-axis, and Z-axis) orthogonal to each other. An origin of the coordinate axis image 402 represents the target relative position of the bone 102. A posture of the coordinate axis image 402 represents the target relative posture of the bone 102.

The coordinate axis image 403 is represented by three line segments (X-axis, Y-axis, and Z-axis) orthogonal to each other. An origin of the coordinate axis image 403 represents the relative position of the bone 102 with respect to the target relative position of the bone 102. A posture of the coordinate axis image 403 represents the relative posture of the bone 102.

The numerical value information representing the difference is, for example, a numerical value representing a distance in millimeters. The character information representing a movement direction is, for example, a character string such as "Left" and "Down". The surgeon may move the bone 102 in the movement direction represented by the character information so that the value of the numerical value information becomes small while checking the navigation image. Further, the distance (proximity) from the target relative position to the relative position and the character information "Proximity" may be displayed. In addition, the numerical value information representing the difference may be a difference between the posture of the coordinate axis image 402 and the posture of the coordinate axis image 403 (match rate for each axis). A matter that the posture of the coordinate axis image 402 matches the posture of the coordinate axis image 403 means that the target relative posture of the bone 102 matches the relative posture of the bone 102.

A color of the X-axis of the coordinate axis image 402 and a color of the X-axis of the coordinate axis image 403 are the same color (for example, red). A color of the Y-axis of the coordinate axis image 402 and a color of the Y-axis of the coordinate axis image 403 are the same color (for example, blue). A color of the Z-axis of the coordinate axis image 402 and a color of the Z-axis of the coordinate axis image 403 are the same color (for example, green). Further, a color of the line image 404 connecting the origin (target relative position) of the coordinate axis image 402 and the origin (relative position of the bone 102 at the current timing) of the coordinate axis image 403 is purple as an example. A length of the line image 404 represents a difference (distance) between the target relative position and the relative position of the bone 102 (maxilla model 112).

FIG. 12 is a diagram illustrating an example of a navigation image indicating a position of a bone and the like in the first embodiment. During the surgery, the surgeon moves and rotates the bone 102 such that the coordinate axis image 402 and the coordinate axis image 403 overlap each other while checking the navigation image displayed in real time on the display unit 66 at a predetermined frame rate.

The diagram on the left side illustrated in FIG. 12(part of the navigation image) represents the position and the posture of the bone 101 (skull model 111) and the position and the posture of the bone 102 (maxilla model 112) at a third timing during the surgery. The diagram on the right side illustrated in FIG. 12 (part of the navigation image) represents the position and the posture of the bone 101 (skull model 111) and the position and the posture of the bone 102 (maxilla model 112) at a fourth timing after the third timing. In the diagram on the right side illustrated in FIG. 12, as a result of the surgeon moving and rotating the bone 102, the coordinate axis image 402 and the coordinate axis image 403 substantially overlap each other. In such a case, the relative position and the relative posture of the bone 102 with respect to the position and the posture of the bone 101 are close to the target relative position and the target relative posture determined in advance in the preoperative planning.

The orientation of the attachment portion 211 of the jig 210 attached to the target attachment position of the bone 101 and the orientation of the attachment portion 221 of the jig 220 attached to the bone 102 rejoined to the bone 101 are the same. In this case, the posture of the magnetic sensor 301-2 inserted into the attachment portion 221 and the posture of the magnetic sensor 301-1 inserted into the attachment portion 211 are the same posture (inclination). Accordingly, a relative positional relationship between the coordinate axis image 402 and the coordinate axis image 403 is displayed in an intuitively understandable manner, and thus the navigation surgery becomes extremely easy.

In addition, since the coordinate axis image 402 and the coordinate axis image 403 are respectively represented by the three-dimensional coordinate axes, the surgeon can intuitively determine whether the relative position and the relative posture of the bone 102 accurately match the target relative position and the target relative posture by the surgeon checking the match between the coordinate axis image 402 and the coordinate axis image 403 in the navigation image. Further, the patient can achieve good occlusion and an aesthetic facial appearance.

Hereinafter, the relative position of the first sensor (magnetic sensor 301-1) with respect to the target attachment position of the first sensor is referred to as the "first relative position". Hereinafter, the relative posture of the first sensor (magnetic sensor 301-1) with respect to the target attachment posture of the first sensor is referred to as the "first relative posture".

Next, an operation example of the navigation device 6 when the magnetic sensor is attached to the bone during the surgery will be described.

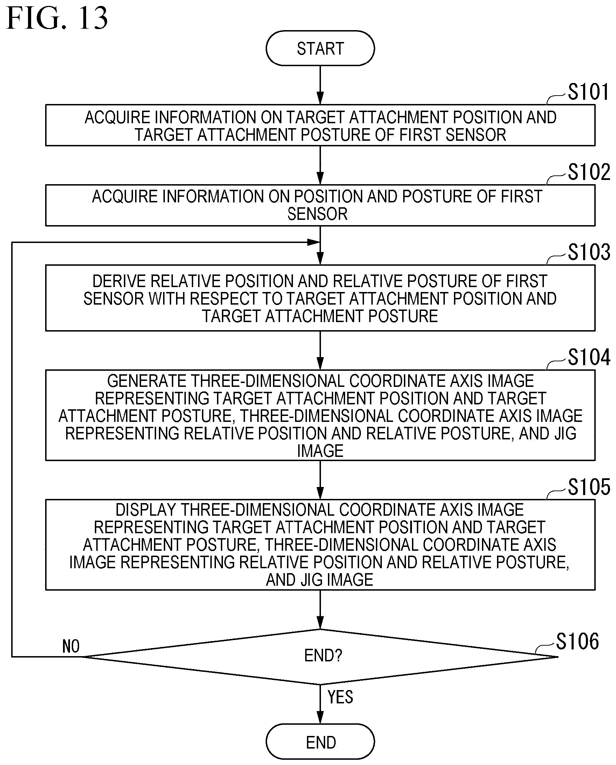

FIG. 13 is a flowchart illustrating a first operation example (operation example when a magnetic sensor is attached to a bone) of the navigation device 6 in the first embodiment. The acquisition unit 63 acquires the information on the target attachment position and the target attachment posture of the magnetic sensor 301-1 with respect to the position and the posture of the magnetic sensor 301-2 from the planning device 2 or the storage device 3 (step S101). The acquisition unit 63 acquires, from the detection device 5, the information on the position and the posture of the magnetic sensor 301-1 detected based on the signal of the magnetic sensor 301-1 (step S102).

The derivation unit 64 derives the first relative position and the first relative posture of the magnetic sensor 301-1 based on the target attachment position and the target attachment posture of the magnetic sensor 301-1 and the position and the posture of the magnetic sensor 301-1 (step S103).

The information generation unit 65 generates the three-dimensional coordinate axis image (coordinate axis image 15) representing the target attachment position and the target attachment posture of the magnetic sensor 301-1. The information generation unit 65 generates a three-dimensional coordinate axis image (coordinate axis image 16) representing the first relative position and the first relative posture of the magnetic sensor 301-1. The information generation unit 65 may generate the jig image 213, the image of the skull model 111, and the image of the maxilla model 112 (step S104). The display unit 66 displays the coordinate axis image 15 and the coordinate axis image 16. The display unit 66 may display the jig image 213, the image of the skull model 111, and the image of the maxilla model 112 (step S105).

The information generation unit 65 determines whether to end the navigation operation. For example, when the surgery ends, the information generation unit 65 determines to end the navigation operation (step S106). When it is determined that the navigation operation is continued (step S106: NO), the information generation unit 65 returns the processing to step S103. When it is determined to end the navigation operation (step S106: YES), the information generation unit 65 ends the navigation operation.

Next, an operation example of the navigation device 6 when the bone is relatively moved and relatively rotated during the surgery will be described.

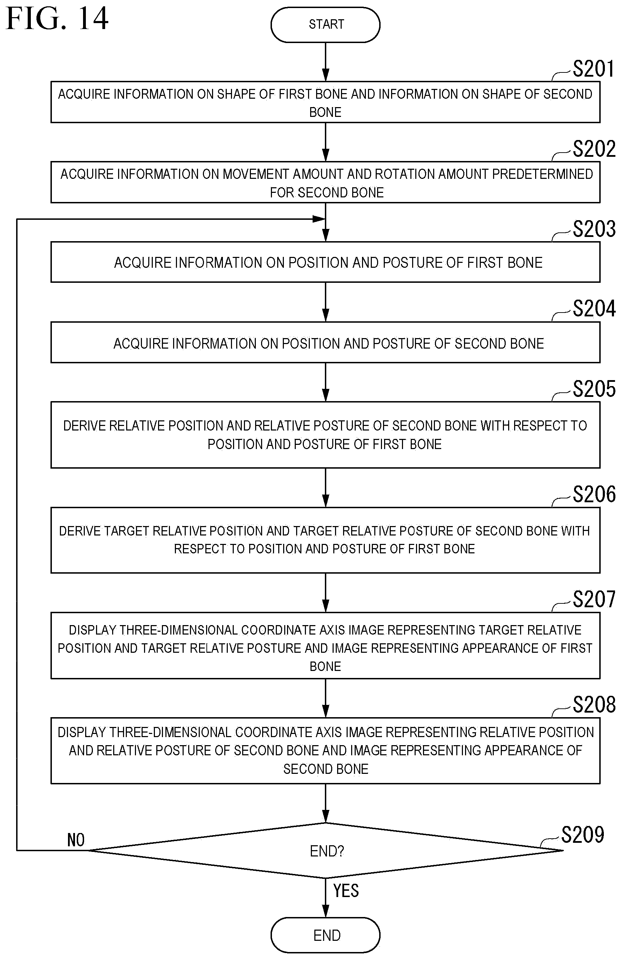

FIG. 14 is a flowchart illustrating a second operation example (operation example when the bone is relatively moved and relatively rotated) of the navigation device 6 in the first embodiment. The acquisition unit 63 acquires the information on the shape of the bone 101 (first bone) and the information on the shape of the bone 102 (second bone) from the communication unit 61 or the storage unit 62 (step S201). The acquisition unit 63 acquires the information on the movement amount and the rotation amount predetermined for the bone 102 from the communication unit 61 or the storage unit 62 (step S202).

During the surgery, after the magnetic sensor 301 is attached to the bone, the communication unit 61 and the storage unit 62 acquire the information on the position and the posture of the bone 101 from the communication unit 54 at a predetermined period. The acquisition unit 63 acquires the information on the position and the posture of the bone 101 from the communication unit 61 or the storage unit 62 at a predetermined period (step S203). The communication unit 61 acquires the information on the position and the posture of the bone 102 from the communication unit 54 at a predetermined period. The acquisition unit 63 acquires the information on the position and the posture of the bone 102 from the communication unit 61 or the storage unit 62 at a predetermined period (step S204).

The derivation unit 64 derives the relative position and the relative posture of the bone 102 with respect to the position and the posture of the bone 101 based on the information on the position and the posture of the bone 101 and the information on the position and the posture of the bone 102 (step S205). The derivation unit 64 derives the target relative position and the target relative posture of the bone 102 with respect to the position and the posture of the bone 101 based on the movement amount and the rotation amount predetermined with respect to the initial values of the relative position and the relative posture of the bone 102 and the relative position and the relative posture of the bone 102 (step S206).

The information generation unit 65 generates predetermined information including the coordinate axis image 402 (three-dimensional coordinate axis image) representing the target relative position and the target relative posture of the bone 102 based on the target relative position and the target relative posture of the bone 102 with respect to the position of the bone 101. The display unit 66 displays, in real time, predetermined information including the coordinate axis image 402 and the image of the skull model 111 representing the appearance of the bone 101 according to the position and the posture of the bone 101 (step S207).

The information generation unit 65 generates predetermined information including the coordinate axis image 403 (three-dimensional coordinate axis image) representing the relative position and the relative posture of the bone 102 based on the relative position and the relative posture of the bone 102 with respect to the position of the bone 101. The display unit 66 displays, in real time, predetermined information including the coordinate axis image 403 and the image of the maxilla model 112 representing the appearance of the bone 102 according to the relative position and the relative posture of the bone 102 (step S208).

The information generation unit 65 determines whether to end the navigation operation. For example, when the surgery ends, the information generation unit 65 determines to end the navigation operation (step S209). When it is determined that the navigation operation is continued (step S209: NO), the information generation unit 65 returns the processing to step S203. When it is determined to end the navigation operation (step S209: YES), the information generation unit 65 ends the navigation operation.

As described above, the detection unit 53 detects the position and the posture of the magnetic sensor 301-1 based on the signal of the magnetic sensor 301-1 (first sensor) inserted into the attachment portion 211 in the first orientation predetermined with respect to the jig 210 which has the joining surface 212 having the surface of the shape corresponding to the surface of the bone 101 (for example, the skull). Further, the detection unit 53 detects the position and the posture of the magnetic sensor 301-2 based on the signal of the magnetic sensor 301-2 (second sensor) inserted into the attachment portion 221 in the second orientation predetermined with respect to the jig 220 which has the joining surface 222 having the surface of the shape corresponding to the surface of the bone 102 (for example, the maxilla).

The acquisition unit 63 acquires, from the detection device 5, the information on the position and the posture of the magnetic sensor 301-1 detected based on the signal of the magnetic sensor 301-1. The acquisition unit 63 acquires the information on the target attachment position and the target attachment posture of the magnetic sensor 301-1 with respect to the position and the posture of the magnetic sensor 301-2 from the planning device 2 or the storage device 3.

The derivation unit 64 derives the first relative position and the first relative posture based on the target attachment position and the target attachment posture, and the position and the posture of the magnetic sensor 301-1. The information generation unit 65 generates the three-dimensional coordinate axis image (coordinate axis image 15) representing the target attachment position and the target attachment posture of the magnetic sensor 301-1. The information generation unit 65 generates the three-dimensional coordinate axis image (coordinate axis image 16) representing the first relative position and the first relative posture of the magnetic sensor 301-1. As illustrated in FIGS. 8 and 9, the display unit 66 displays the coordinate axis image 15 and the coordinate axis image 16.

As described above, the surgeon accurately attaches the magnetic sensor 301-1 in the target attachment posture to the target attachment position in the actual bone 101 while viewing the coordinate axis image 15 representing the target attachment position and the target attachment posture of the magnetic sensor 301-1 in the skull model 111 and the coordinate axis image 16. Accordingly, it is possible to support the surgeon to accurately move and rotate the bone 102 such that the relative position and the relative posture of the bone 102 become the target relative position and the target relative posture.

The information generation unit 65 may generate numerical value information representing a difference between the target attachment position and the first relative position. The display unit 66 may display the numerical value information as illustrated in FIGS. 8 and 9. The information generation unit 65 may generate character information representing a movement direction in which the difference between the target attachment position and the first relative position is reduced. The display unit 66 may display the character information as illustrated in FIGS. 8 and 9. The information generation unit 65 may generate an image of a line connecting the target attachment position and the first relative position. The display unit 66 may display the line image 17 as illustrated in FIGS. 8 and 9.

Hereinafter, the relative position of the second bone (bone 102) with respect to the position of the first bone (bone 101) is referred to as the "second relative position". Hereinafter, the relative posture of the second bone with respect to the posture of the first bone is referred to as the "second relative posture".

The acquisition unit 63 acquires, from the detection device 5, the information on the position and the posture of the bone 101 detected based on the signal of the magnetic sensor 301-1 attached to the bone 101 (for example, the skull). The acquisition unit 63 acquires, from the detection device 5, the information on the position and the posture of the bone 102 detected based on the signal of the magnetic sensor 301-2 attached to the bone 102 (for example, the maxilla).

The derivation unit 64 derives the second relative position and the second relative posture of the bone 102 with respect to the position of the bone 101 based on the information on the position and the posture of the bone 101 and the information on the position and the posture of the bone 102. The derivation unit 64 derives the target relative position and the target relative posture of the bone 102 with respect to the position and the posture of the bone 101 based on a movement amount predetermined with respect to an initial value of the second relative position and a rotation amount predetermined with respect to an initial value of the second relative posture.

The information generation unit 65 generates the three-dimensional coordinate axis image (coordinate axis image 402) representing the target relative position and the target relative posture. The information generation unit 65 generates the three-dimensional coordinate axis image (coordinate axis image 403) representing the second relative position and the second relative posture. As illustrated in FIGS. 11 and 12, the display unit 66 displays the coordinate axis image 402 and the coordinate axis image 403.

Accordingly, since the movement state and the rotation state of the bone 102 are shown to the surgeon, it is possible to support the surgeon to accurately move and rotate the bone 102 to achieve the predetermined target relative position and target relative posture.

### (Second Embodiment)

In a second embodiment, a difference from the first embodiment is that a magnetic sensor (jig) is also attached to a mandible proximal bone fragment separated from the mandible. In the second embodiment, the difference from the first embodiment will be mainly described.

### <Before Surgery>

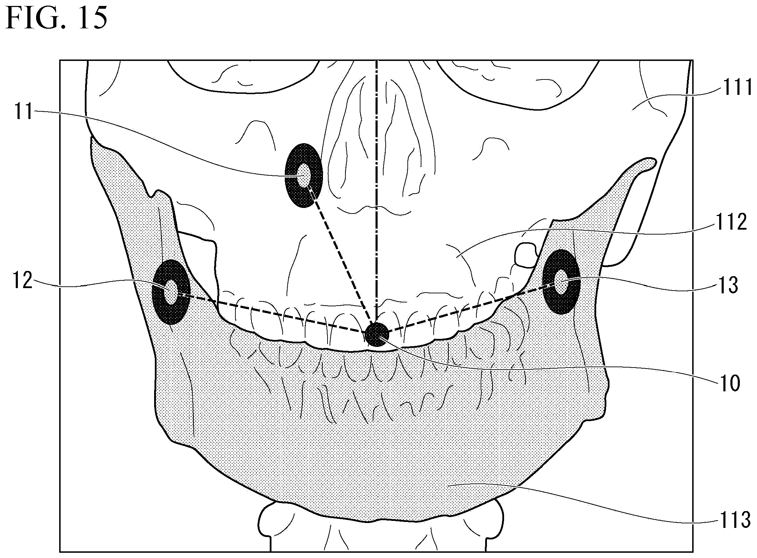

FIG. 15 is a diagram illustrating a first example of designation of attachment positions of the magnetic sensors 301 in the second embodiment. The attachment positions of the magnetic sensors 301 are illustrated on the screen of the display unit 23 in the state before the maxilla model 112 is separated from the skull model 111. The mandible model 113 is a three-dimensional model of the mandible of the patient.

In FIG. 15, in the preoperative planning, the jig 220 illustrated in FIG. 1 is sandwiched between and joined to the dentition of the maxilla model 112 and the dentition of the mandible model 113. The attachment position 10 represents the attachment position of the magnetic sensor 301-2 (jig 220) actually attached to the bone 102. The attachment position 11 represents the target attachment position of the magnetic sensor 301-1 actually attached to the bone 101. An attachment position 12 and an attachment position 13 represent the target attachment positions of the magnetic sensors 301 attached to the actual mandible. The attachment position 11, the attachment position 12, and the attachment position 13 are expressed with the attachment position 10 as the origin.

### <During Surgery>

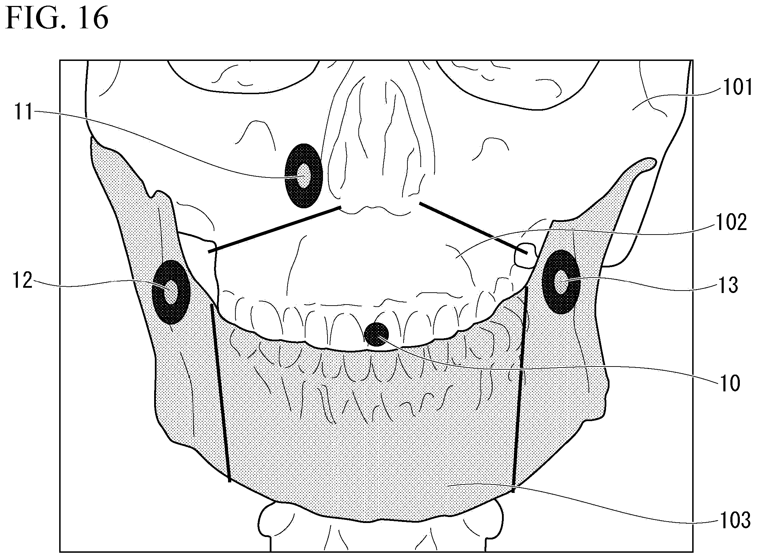

FIG. 16 is a diagram illustrating an example of cutting a bone 103 (mandible) in the second embodiment. During the surgery, the surgeon temporarily removes the magnetic sensors 301 from the bones. The surgeon separates the bone 102 from the bone 101 and cuts the bone 103.

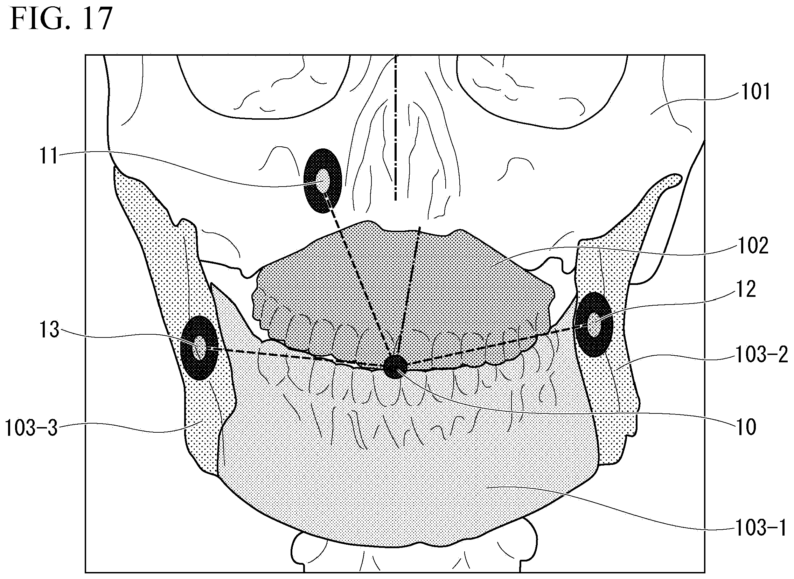

FIG. 17 is a diagram illustrating an example of the attachment positions of the magnetic sensors 301 after cutting the bone 103 in the second embodiment. The attachment positions of the magnetic sensors 301 are illustrated in a state where the bone 102 is separated from the bone 101 and the bone 102 is rejoined to the bone 101.

In FIG. 17, the bone 103 (mandible) is cut into a bone 103-1, a bone 103-2 (mandible proximal bone fragment), and a bone 103-3 (mandible proximal bone fragment). In the maxilla model 112 and the mandible model 113-1, the attachment position 10 of the first magnetic sensor 301-1 is determined by the planner. In the skull model 111, the attachment position 11 of the second magnetic sensor 301-2 is determined by the planner. In the mandible model 113-2, the attachment position 12 of the third magnetic sensor 301-3 is determined by the planner. In the mandible model 113-3, the attachment position 13 of the fourth magnetic sensor 301-4 is determined by the planner. The attachment position 11, the attachment position 12, and the attachment position 13 are expressed with the attachment position 10 as the origin.

Accordingly, for example, regarding the mandible proximal bone fragment of the bone 103, it is also possible to support the surgeon to accurately move and rotate the bone 103 such that a relative position and a relative posture of the bone 103 become the target relative position and the target relative posture.

The second embodiment is suitable for surgery in which alignment of a plurality of bones is performed (for example, surgery to reconstruct an artificial bone in a patient who has lost bone due to osteonecrosis of the jaw caused by cancer) .

As described above, the embodiments of the invention has been described in detail with reference to the drawings, specific configurations are not limited to the embodiments, and designs and the like within a range not departing from the gist of the invention are also included.

For example, the number of sensors is not limited to a specific number. For example, by executing navigation using three or more sensors, the surgeon can more accurately adjust a relative position and a relative posture of a bone.

The navigation surgery in the embodiments is not limited to surgery in a specific field as long as the surgery requires an accurate match of relative positions and the relative postures of bones. For example, the navigation surgery may be navigation surgery in orthognathic surgery between a maxilla and a mandible. Here, a jig (mouthpiece) for the maxilla and a jig (mouthpiece) for the mandible may be produced. A sensor may be attached to each of the jigs.

### Industrial Applicability

The invention is applicable to a surgery support system.

### Reference Signs List

1 navigation system
2 planning device
3 storage device
4 communication line
5 detection device
6 navigation device
10 attachment position
11 attachment position
12 attachment position
13 attachment position
14 coordinate axis image
15 coordinate axis image
16 coordinate axis image
17 line image
18 display region
21 movement processing unit
22 rotation processing unit
23 display unit
51 magnetic field generation unit
52 acquisition unit
53 detection unit
54 communication unit
61 communication unit
62 storage unit
63 acquisition unit
64 derivation unit
65 information generation unit
66 display unit
101 bone
102 bone
103 bone
111 skull model
112 maxilla model
113 mandible model
210 jig
211 attachment portion
212 joining surface
213 jig image
220 jig
221 attachment portion
222 joining surface
223 jig image
301 magnetic sensor
401 display region
402 coordinate axis image
403 coordinate axis image
404 line image

## Claims

1. A navigation system comprising:
an acquisition unit configured to acquire information on a position and a posture of a first sensor detected based on a signal of the first sensor, and information on a target attachment position and a target attachment posture of the first sensor with respect to a position and a posture of a second sensor detected based on a signal of the second sensor, the first sensor being inserted into an attachment portion of a first jig, which has a surface having a shape corresponding to a surface of a first bone, in a first orientation predetermined with respect to the first jig, and the second sensor being inserted into an attachment portion of a second jig, which has a surface having a shape corresponding to a surface of a second bone, in a second orientation predetermined with respect to the second jig;
a derivation unit configured to derive a first relative position and a first relative posture of the first sensor with respect to the target attachment position and the target attachment posture based on the target attachment position, the target attachment posture, and the position and the posture of the first sensor;
an information generation unit configured to generate a three-dimensional coordinate axis image representing the target attachment position and the target attachment posture and a three-dimensional coordinate axis image representing the first relative position and the first relative posture; and
a display unit configured to display the three-dimensional coordinate axis image representing the target attachment position and the target attachment posture and the three-dimensional coordinate axis image representing the first relative position and the first relative posture.

2. The navigation system according to claim 1, wherein
the second jig is a dental splint.

3. The navigation system according to claim 1, wherein
the information generation unit generates numerical value information representing a difference between the target attachment position and the first relative position, and
the display unit further displays the numerical value information.

4. The navigation system according to claim 1, wherein
the information generation unit generates character information representing a movement direction in which a difference between the target attachment position and the first relative position is reduced, and
the display unit further displays the character information.

5. The navigation system according to claim 1, wherein
the information generation unit generates an image of a line connecting the target attachment position and the first relative position, and
the display unit further displays the image of the line.

6. The navigation system according to claim 1, wherein
the sensors are magnetic sensors.

7. The navigation system according to claim 1, wherein
the first bone is a skull, and
the second bone is a maxilla.

8. The navigation system according to claim 1, wherein
the acquisition unit acquires information on a position and a posture of the first bone detected based on the signal of the first sensor attached to the first bone and information on a position and a posture of the second bone detected based on the signal of the second sensor attached to the second bone,
the derivation unit derives a second relative position and a second relative posture of the second bone with respect to the position of the first bone based on the information on the position and the posture of the first bone and the information on the position and the posture of the second bone, and derives a target relative position and a target relative posture of the second bone with respect to the position and the posture of the first bone based on a movement amount predetermined with respect to an initial value of the second relative position and a rotation amount predetermined with respect to an initial value of the second relative posture,
the information generation unit generates a three-dimensional coordinate axis image representing the target relative position and the target relative posture and a three-dimensional coordinate axis image representing the second relative position and the second relative posture, and
the display unit displays the three-dimensional coordinate axis image representing the target relative position and the target relative posture and the three-dimensional coordinate axis image representing the second relative position and the second relative posture.

9. A navigation system comprising:
an acquisition unit configured to acquire information on a position and a posture of a first bone detected based on a signal of a first sensor, and information on a position and a posture of a second bone detected based on a signal of a second sensor, the first sensor being inserted into an attachment portion of a first jig, which has a surface having a shape corresponding to a surface of the first bone, in a first orientation predetermined with respect to the first jig, and the second sensor being inserted into an attachment portion of a second jig, which has a surface having a shape corresponding to a surface of the second bone, in a second orientation predetermined with respect to the second jig;
a derivation unit configured to derive a relative position and a relative posture of the second bone with respect to the position of the first bone based on the information on the position and the posture of the first bone and the information on the position and the posture of the second bone, and derive a target relative position and a target relative posture of the second bone with respect to the position and the posture of the first bone based on a movement amount predetermined with respect to an initial value of the relative position and a rotation amount predetermined with respect to an initial value of the relative posture;
an information generation unit configured to generate a three-dimensional coordinate axis image representing the target relative position and the target relative posture and a three-dimensional coordinate axis image representing the relative position and the relative posture; and
a display unit configured to display the three-dimensional coordinate axis image representing the target relative position and the target relative posture and the three-dimensional coordinate axis image representing the relative position and the relative posture.

10. A jig comprising:
an attachment portion configured to allow a sensor to be inserted in a predetermined orientation; and
a surface having a shape corresponding to a surface of a bone to be joined.

11. The jig according to claim 10, comprising:
a first of the jigs including a first of the attachment portions configured to allow a first of the sensors to be inserted in a first orientation predetermined with respect to the first of the jigs, and a first of the surfaces having a shape corresponding to a surface of a first of the bones; and
a second of the jigs including a second of the attachment portions configured to allow a second of the sensors to be inserted in a second orientation predetermined with respect to the second of the jigs, and a second of the surfaces having a shape corresponding to a surface of a second of the bones, wherein
the orientation of the first of the sensors inserted into the first of the attachment portions of the first of the jigs while being joined to the first of the bones and the orientation of the second of the sensors inserted into the second of the attachment portions of the second of the jigs while being joined to the second of the bones are the same orientation.

12. A program for causing a computer to execute steps of:
acquiring information on a position and a posture of a first sensor detected based on a signal of the first sensor, and information on a target attachment position and a target attachment posture of the first sensor with respect to a position and a posture of a second sensor detected based on a signal of the second sensor, the first sensor being inserted into an attachment portion of a first jig, which has a surface having a shape corresponding to a surface of a first bone, in a first orientation predetermined with respect to the first jig, and the second sensor being inserted into an attachment portion of a second jig, which has a surface having a shape corresponding to a surface of a second bone, in a second orientation predetermined with respect to the second jig;
deriving a first relative position and a first relative posture of the first sensor with respect to the target attachment position and the target attachment posture based on the target attachment position, the target attachment posture, and the position and the posture of the first sensor;
generating a three-dimensional coordinate axis image representing the target attachment position and the target attachment posture and a three-dimensional coordinate axis image representing the first relative position and the first relative posture; and
displaying the three-dimensional coordinate axis image representing the target attachment position and the target attachment posture and the three-dimensional coordinate axis image representing the first relative position and the first relative posture.

13. A program for causing a computer to execute steps of:
acquiring information on a position and a posture of a first bone detected based on a signal of a first sensor, and information on a position and a posture of a second bone detected based on a signal of a second sensor, the first sensor being inserted into an attachment portion of a first jig, which has a surface having a shape corresponding to a surface of the first bone, in a first orientation predetermined with respect to the first jig, and the second sensor being inserted into an attachment portion of a second jig, which has a surface having a shape corresponding to a surface of the second bone, in a second orientation predetermined with respect to the second jig;
deriving a relative position and a relative posture of the second bone with respect to the position of the first bone based on the information on the position and the posture of the first bone and the information on the position and the posture of the second bone, and deriving a target relative position and a target relative posture of the second bone with respect to the position and the posture of the first bone based on a movement amount predetermined with respect to an initial value of the relative position and a rotation amount predetermined with respect to an initial value of the relative posture;
generating a three-dimensional coordinate axis image representing the target relative position and the target relative posture and a three-dimensional coordinate axis image representing the relative position and the relative posture; and
displaying the three-dimensional coordinate axis image representing the target relative position and the target relative posture and the three-dimensional coordinate axis image representing the relative position and the relative posture.
